# FASCICULE DE BREVET EUROPEEN

(11) **EP 0 775 488 B1**
(45) Date de publication et mention de la délivrance du brevet: **12.10.2005**
(21) Numéro de dépôt: 96402398.0
(22) Date de dépôt: 12.11.1996
(51) Int. Cl.: A61K 31/355, A61K 31/616, A61K 9/48

(54) **Composition pharmaceutique stable à base d'acide acétylsalicyclique et de tocophérol**
Stabile pharmazeutische Zubereitung auf Basis von Acetylsalicylsäure und Tocopherol
Stable pharmaceutical composition on the basis of acetylsalicyclic acid and tocopherol

(30) Priorité: 23.11.1995 FR 9513933
(43) Date de publication de la demande: 28.05.1997
(73) Titulaire: Mazal Pharmaceutique, 29000 Quimper (FR)
(72) Inventeur: Estanove, Cyril, 92100 Boulogne (FR); Prudhomme, Alain, 29940 La Foret Fouesnant (FR)
(74) Mandataire: L'Helgoualch, Jean

(56) Documents cités:
- EP-A- 0 204 987
- EP-A- 0 669 132
- WO-A-84/00004
- FR-A- 2 186 219
- US-A- 3 341 416
- US-A- 3 488 418
- 1981, HAWLEY'S CONDENSED CHEMICAL DICTIONNARY, ELEVENTH EDITION, ASPIRIN * page 103 *

## Description

La présente invention concerne une nouvelle composition pharmaceutique stable à base de tocophérol et d'acide acétylsalicylique, et plus particulièrement une nouvelle composition présentant une excellente stabilité et permettant l'administration d'acide acétylsalicylique par voie orale en une seule prise quotidienne, comme médicament pour la prévention des maladies cardio-vasculaires et comme adjuvant en chimiothérapie des cancers.

L'acide acétylsalicylique, ou aspirine, est un composé bien connu en thérapeutique où il est utilisé comme principe actif, isolément ou en combinaison, dans divers médicaments en raison de ses propriétés pharmacologiques, et notamment de ses propriétés antipyrétiques, analgésiques et anti-inflammatoires. On sait également que l'aspirine possède une activité antithrombotique utile dans la prévention de l'infarctus du myocarde si elle est administrée régulièrement à des doses de l'ordre de 100 mg à 1 g par jour.

Toutefois, l'utilisation de l'aspirine s'accompagne souvent d'effets secondaires gênants tels que des irritations gastro-intestinales, des hémorragies ou des allergies.

L'acide acétylsalicylique peut être préparé sous forme utilisable dans le domaine pharmaceutique par divers procédés connus, par exemple par les procédés décrits dans les brevets US-A-2.890.240 et US-A-3.235.583. Il peut être obtenu sous diverses formes, par exemple micropulvérisée, granulée, cristallisée cubique ou aciculaire.

Le tocophérol, et en particulier la vitamine E (α-tocophérol) possède des propriétés antioxydantes bien connues. Les formes les plus courantes du tocophérol sont l'α, le β-, le γ- et le δ-tocophérol, qui sont des dérivés tétra- ou triméthylés du chromanol ne différant que par la présence et la position de groupes méthyle sur le noyau chromanol de la molécule. Chacun de ces tocophérols peut se trouver sous les formes d, l ou dl. Ces tocophérols et leurs propriétés antioxydantes sont décrits par exemple dans le brevet US-A-2.486.541.

On sait que divers essais ont été faits pour associer la vitamine E à l'acide acétylsalicylique afin de protéger l'organisme et limiter ou masquer les effets secondaires de l'aspirine. Ainsi, le brevet FR-A-2.314.722 décrit des esters constitués par des acétylsalicylates de tocophérol, présentés comme possédant une activité anti-agrégante plaquettaire. Le brevet US-A-4.454.112 décrit des propriétés de ces mêmes composés permettant d'envisager leur utilisation comme agents antisolaires.

D'autre part, les effets de l'utilisation de substances antioxydantes liposolubles telles que la vitamine A et la vitamine E pour protéger la muqueuse gastrique contre l'action de l'acide acétylsalicylique, ont aussi été étudiés chez le rat [J. Tissue React. n°8, pp. 35-40 (1986)]. On sait par ailleurs que la vitamine E agit comme piège pour les radicaux libres et limite ainsi l'oxydation des protéines de basse densité (LDL) dont le rôle est important dans l'étiologie de l'athérosclérose.

La demande de brevet EP-A-669.132 indique que l'administration d'acide acétylsalicylique et de vitamine E est utile pour le traitement de l'athérosclérose, mais elle ne décrit aucun exemple de composition.

Enfin, le brevet EP-B-204.987 décrit l'utilisation de doses élevées de vitamine E pour améliorer les propriétés d'écoulement du sang en présence de diverses substances, telles que des vitamines A, B ou C, des analgésiques. L'acide acétylsalicylique peut être utilisé comme analgésique en association avec la vitamine E, les deux composés étant rigoureusement séparés dans une forme galénique adaptée. Cependant, aucune indication n'est donnée quant à la stabilité de cette association.

Il est donc souhaitable de pouvoir mettre au point une composition associant de manière stable le tocophérol et l'acide acétylsalicylique de manière à en faciliter l'administration, de préférence par voie orale.

Cependant, la mise au point d'une telle association se heurte à d'importantes difficultés techniques car l'acide acétylsalicylique et le tocophérol sont des composés très différents, tant par leurs propriétés chimiques que par leurs propriétés physiques. Plus particulièrement, l'acide acétylsalicylique est un composé solide polaire et instable, tandis que l'acétate de tocophérol, utilisé en pharmacie de préférence au tocophérol libre qui est instable, est un liquide huileux apolaire. En particulier, l'acide acétylsalicylique se dégrade rapidement en acide salicylique et acide acétique en présence d'eau.

Ainsi, lorsque l'on veut administrer simultanément 125 mg d'acide acétylsalicylique et 250 mg d'acétate de tocophérol, suivant les techniques classiques, sous forme de gélules ou de comprimés, soit 375 mg de principes actifs au total, il est tout d'abord nécessaire de fixer l'acétate de tocophérol sur un support. Pour une capacité de fixation de 50% (m/m), ceci porte la masse totale à 625 mg, pour les deux principes actifs, et il faut encore incorporer à cette masse une charge d'excipient (dans le cas d'une forme pharmaceutique nécessitant une compression) représentant 30 à 40% en poids, d'où il résulte une masse de l'ensemble d'au moins 810 mg. Une telle quantité peut très difficilement être administrée sous forme de gélule ou de comprimé en une seule prise par voie orale.

La présente invention a donc pour objet une nouvelle composition pharmaceutique stable à base d'acide acétylsalicylique et de tocophérol, adaptée à une administration par voie orale à raison d'une seule prise quotidienne, pour le traitement ou la prévention des maladies cardio-vasculaires ou pour le traitement d'appoint en chimiothérapie des cancers afin de limiter les effets secondaires des principes actifs utilisés dans le traitement.

La composition conforme à la présente invention est adaptée pour l'administration par voie orale, et se distingue en ce qu'elle comprend des capsules suivant la revendication 1.

La composition suivant la présente invention permet donc d'associer de manière stable un liquide huileux tel qu'un tocophérol, et de préférence l'acétate d'α-tocophérol, et un solide, l'aspirine, et présente l'avantage de permettre l'administration des deux composés qui peuvent être présents en quantités relativement importantes, par exemple 20 à 300 mg d'acide acétylsalicylique pour 20 à 600 mg de tocophérol par capsule, sans qu'il soit nécessaire de recourir à un support usuel pour fixer le tocophérol. La composition de l'invention est donc particulièrement avantageuse en ce qu'elle permet l'administration de l'aspirine et du tocophérol en une seule prise quotidienne. Bien entendu, selon la prescription du médecin, l'administration peut être faite dans certains cas en deux prises quotidiennes sans sortir du cadre de l'invention.

De plus, la composition présente une excellente stabilité à la conservation. On sait que l'acide acétylsalicylique se dégrade fortement au contact de la gélatine, principalement en milieu humide. Or, on a observé, de manière inattendue, que la stabilité de la composition de l'invention se trouvait encore améliorée par l'effet protecteur procuré par le tocophérol sous forme huileuse dans la capsule, en particulier dans le cas d'une capsule gélatineuse, même en présence de glycérol, comme indiqué ci-après.

Le tocophérol utilisé dans la composition de l'invention peut être l'α, le β-, le γ- ou le δ-tocophérol, sous forme d, l ou dl. En raison de l'instabilité bien connue du tocophérol libre, il est préférable d'utiliser un ester ou un sel pharmaceutiquement acceptable, tel que l'acétate, qui se présente sous forme d'huile. Dans la présente invention, on utilise de préférence l'acétate d'α-tocophérol, sous forme d, l ou dl.

L'acide acétylsalicylique utilisé comme composant dans la composition de l'invention est sous forme cristalline ou pulvérisée, et la granulométrie des grains ou cristaux d'acide acétylsalicylique est de préférence inférieure à 500 µm. D'excellents résultats ont été obtenus avec de l'acide acétylsalicylique cristallisé avec une granulométrie d'environ 300 µm, mais cette valeur ne constitue pas une limite, et de bons résultats sont également obtenus avec, par exemple, de l'acide acétylsalicylique pulvérisé ou micropulvérisé dont la granulométrie est inférieure à 90 µm.

Suivant une forme préférentielle de réalisation de l'invention, les grains d'acide acétylsalicylique sont encapsulés dans un dérivé cellulosique, tel qu'un hydroxyalkyl éther ou un alkyl éther de cellulose de haut poids moléculaire, seul ou en combinaison, et de préférence l'éthyl cellulose.

Les capsules contenant la composition suivant la présente invention sont des capsules dures ou des capsules molles d'un type connu dans la technique pharmaceutique, et de préférence des capsules molles à base de gélatine et d'autres excipients en proportions variables, tels que le glycérol ou le sorbitol, ou des dérivés de silicone, ou à base de cellulose ou d'amidon modifié.

On peut ainsi disposer de capsules ne contenant que les deux principes actifs, c'est-à-dire l'acide acétylsalicylique et le tocophérol, sans additif ni support, et cette composition présente une excellente stabilité, comme indiqué ci-après, malgré les différences de propriétés physico-chimiques des deux composants.

Toutefois, afin de faciliter le remplissage des capsules, au cours de la fabrication, il peut être avantageux de diminuer la viscosité de la masse en ajoutant des excipients pharmaceutiquement acceptables. Ces excipients ont pour but d'ajuster la viscosité de la composition pour faciliter le remplissage des capsules, et ils doivent être inertes vis-à-vis des principes actifs utilisés dans l'invention, et être miscibles avec le tocophérol lorsqu'il est utilisé sous forme d'acétate huileux. On peut utiliser en particulier des excipients choisis parmi des triglycérides saturés et des triglycérides à chaînes moyennes, par exemple le Miglyol®, ou des mélanges de paraffines aliphatiques ou ramifiées, de naphtènes et de dérivés aromatiques, par exemple des vaselines, ces excipients étant utilisés seuls ou en combinaison.

Les études effectuées ont montré que la stabilité de la composition dans le temps n'est pas modifiée par l'addition de ces excipients, qui présentent en outre l'avantage d'éviter la sédimentation de l'acide acétylsalicylique et sa dégradation. Au contraire, dans le cas de capsules contenant uniquement de l'acide acétylsalicylique, ou de l'acide acétylsalicylique associé à des excipients, sans tocophérol, on observe rapidement une dégradation de l'acide acétylsalicylique qui se traduit par la formation d'acide salicylique et d'acide acétique. Ceci montre bien l'effet protecteur assuré par le tocophérol dans la composition de l'invention.

Il est important de noter que les capsules contenant la composition à base d'acide acétylsalicylique et de tocophérol suivant la présente invention présentent une excellente stabilité, même en l'absence d'excipient, et même lorsque la paroi des capsules est à base de gélatine, de glycérol ou de produit similaire susceptible de retenir ou de former des traces d'eau qui peuvent entraîner la dégradation de l'acide acétylsalicylique.

Des excipients usuels peuvent être utilisés, le cas échéant, pour la fabrication de la tunique de la capsule. On peut par exemple utiliser un mélange de gélatine et de plastifiant (tel que le glycérol ou le sorbitol) en proportions variables.

Divers additifs usuels peuvent être ajoutés dans la paroi des capsules pour en modifier les caractéristiques physico-chimiques, tels que des silicones, des conservateurs, des colorants ou des agents opacifiants. On peut utiliser par exemple une silicone ou un polysiloxane tel que la diméthicone ou l'Anhydrisorb®, pour opacifier la paroi.

Les capsules sont de préférence conditionnées sous des emballages d'un type couramment utilisé dans le domaine pharmaceutique, et par exemple un composite aluminium / chlorure de polyvinyle (PVC) ou aluminium / PVDC-PVC / PVDC (chlorure de polyvinylidène), ou un polyéthylène haute densité, un polypropylène en association avec l'aluminium.

La composition selon la présente invention est peu toxique, sa toxicité étant au maximum celle de l'acide acétylsalicylique, qui peut être limitée aux problèmes d'allergie et à la formation d'ulcères gastro-duodénaux. De plus, dans la composition suivant l'invention, l'association de l'aspirine avec le tocophérol permet de réduire la toxicité liée à l'ulcération des muqueuses digestives en raison de l'effet anti-oxydant du tocophérol (vitamine E).

La composition suivant l'invention possède des propriétés anti-agrégantes plaquettaires analogues à celles de l'aspirine. De plus, ces propriétés sont potentialisées par l'action du tocophérol au niveau du métabolisme des prostaglandines des réticulocytes. On peut ainsi diminuer les doses d'acide acétylsalicylique nécessaires pour obtenir une même activité anti-agrégante plaquettaire par rapport à l'acide acétylsalicylique seul, ce qui réduit encore la toxicité de la composition de l'invention.

Ainsi, la composition de l'invention peut être utilisée pour la prévention primaire, notamment dans la prophylaxie des affections cardio-vasculaires, et en particulier pour la prévention de l'infarctus du myocarde. Elle peut être également utilisée pour la prévention secondaire, notamment des affections cardiaques, par exemple dans la prévention des récidives de l'infarctus du myocarde, mais aussi dans le traitement des cancers, par les effets anti-oxydants, piégeurs de radicaux libres, du tocophérol (vitamine E) qui peuvent corriger les effets secondaires de produits anticancéreux caractérisés par leur cytotoxicité cellulaire.

Des exemples de composition conforme à la présente invention sont donnés ci-après.

### Exemple 1

On prépare une composition contenant 125 mg d'acide acétylsalicylique et 250 mg d'acétate d'α-tocophérol par capsule comme indiqué ci-après.

L'acide acétylsalicylique utilisé est sous forme cristallisée, de granulométrie moyenne égale à environ 300 µm, les cristaux étant enrobés par de l'éthylcellulose.

La tunique des capsules est préparée suivant une technique classique, en solubilisant de la gélatine dans de l'eau (54% m/m) et en ajoutant les excipients (sorbitol + sorbitans : 33% m/m; diméthicone : 13% m/m) jusqu'à obtention d'une masse homogène.

D'autre part, on prépare la composition de l'invention en mélangeant les principes actifs et les excipients indiqués ci-après :

| | |
|---|---|
| acide acétylsalicylique cristallisé encapsulé | 132,0 mg |
| acétate d'α-(dl)-tocophérol | 250,0 mg |
| triglycérides saturés | 85,0 mg |
| triglycérides à chaîne moyenne | 33,0 mg |

Les quantités indiquées ci-dessus correspondent à une capsule. La quantité de 132 mg d'acide acétylsalicylique enrobé d'éthylcellulose correspond à 125 mg d'acide.

Après un lissage de la composition suivant les techniques usuelles pour améliorer l'homogénéité, on procède au remplissage en maintenant la masse de la composition sous agitation, pour éviter la sédimentation du produit et assurer l'uniformité des teneurs en principes actifs des capsules.

Les capsules sont ensuite séchées et conditionnées.

Pour effectuer des essais de stabilité à 25°C sous humidité ambiante, un premier lot est conditionné en utilisant un conditionnement aluminium/PVC (chlorure de polyvinyle), et un deuxième lot avec un conditionnement complexe aluminium/PVDC - PVC/PVDC (chlorure de polyvinylidène).

Le tableau ci-après indique les valeurs moyennes des teneurs en acide salicylique et en acide acétylsalicylique au cours de l'essai de stabilité à 25°C.

| Temps en mois | Al./PVC | | Al./PVDC- PVC/PVDC | |
|---|---|---|---|---|
| | Ac. acétylsal. | Ac. salicyl. | Ac. acétylsal. | Ac. salicyl. |
| 0 | 24,8 | 0,28 | 24,2 | 0,31 |
| 3 | 24,8 | 0,78 | 24,5 | 1,00 |
| 6 | 24,7 | 0,80 | 24,5 | 1,29 |
| 12 | 25,4 | 0,94 | 24,5 | 1,56 |
| 18 | 24,5 | 1,09 | 24,2 | 1,78 |
| 24 | 24,8 | 1,26 | 24,3 | 1,98 |
| 30 | 25,3 | 1,39 | 24,8 | 2,27 |
| 36 | 23,4 | 2,17 | 23,0 | 3,00 |

Ces résultats confirment l'excellente stabilité de la composition suivant la présente invention puisque le taux d'acide salicylique reste très faible, indiquant une faible dégradation de l'acide acétylsalicylique.

En particulier, le taux de dégradation est nettement inférieur aux teneurs limites reconnues suivant les normes USP XXIII.

De même, la dégradation de l'acétate de tocophérol en tocophérol libre est extrêmement faible, comme le montrent les résultats suivants, correspondant à l'analyse des mêmes lots conservés dans les mêmes conditions.

| Temps en mois | Al./PVC | | Al./PVDC- PVC/PVDC | |
|---|---|---|---|---|
| | Acétate toco. | Toco. libre | Acétate toco. | Toco. libre |
| 0 | 50,0 | 0,20 | 49,7 | 0,20 |
| 3 | 50,9 | 0,24 | 51,5 | 0,22 |
| 6 | 49,4 | 0,24 | 49,2 | 0,25 |
| 12 | 50,0 | 0,40 | 50,6 | 0,57 |
| 18 | 50,0 | 0,39 | 50,0 | 0,48 |
| 24 | 49,6 | 0,39 | 49,5 | 0,51 |
| 30 | 50,5 | 0,53 | 51,0 | 0,63 |
| 36 | 48,0 | 0,84 | 48,1 | 0,82 |

### Exemple 2

On procède comme dans l'Exemple 1, mais en remplaçant l'acide acétylsalicylique cristallisé par de l'acide acétylsalicylique micropulvérisé (non enrobé) de granulométrie moyenne égale à 90 µm.

Les essais de stabilité à trois ans, effectués dans les mêmes conditions que dans l'Exemple 1, procurent les résultats indiqués ci-après pour la stabilité de l'acide acétylsalicylique.

Les résultats indiqués au tableau ci-après montrent une excellente stabilité, en particulier dans le cas du conditionnement aluminium / PVC.

| Temps en mois | Al./PVC | | Al./PVDC- PVC/PVDC | |
|---|---|---|---|---|
| | Ac. acétylsal. | Ac. salicyl. | Ac. acétylsal. | Ac. salicyl. |
| 0 | 24,2 | 0,22 | 24,0 | 0,25 |
| 3 | 24,3 | 1,01 | 24,6 | 1,21 |
| 6 | 25,0 | 1,14 | 24,9 | 1,50 |
| 12 | 25,0 | 1,52 | 24,8 | 2,21 |
| 18 | 24,0 | 2,01 | 23,6 | 2,97 |
| 24 | 23,7 | 2,52 | 23,6 | 3,76 |
| 30 | 24,0 | 3,58 | 23,8 | 4,90 |
| 36 | 22,8 | 4,93 | 22,2 | 6,17 |

Les résultats en ce qui concerne la stabilité de l'acétate de tocophérol sont les suivants.

| Temps en mois | Al./PVC | | Al./PVDC- PVC/PVDC | |
|---|---|---|---|---|
| | Acétate toco. | Toco. libre | Acétate toco. | Toco. libre |
| 0 | 49,6 | 0,19 | 49,9 | 0,19 |
| 3 | 51,2 | 0,24 | 49,8 | 0,24 |
| 6 | 48,9 | 0,24 | 49,6 | 0,27 |
| 12 | 50,1 | 0,40 | 49,8 | 0,53 |
| 18 | 50,7 | 0,44 | 50,3 | 0,59 |
| 24 | 50,0 | 0,51 | 49,1 | 0,72 |
| 30 | 50,4 | 0,71 | 50,1 | 0,91 |
| 36 | 47,7 | 1,17 | 47,0 | 1,12 |

### Exemple 3

On prépare une composition contenant 30 mg d'acide acétylsalicylique et 30 mg d'acétate d'α-(dl)-tocophérol par capsule.

L'acide acétylsalicylique utilisé est identique à celui de l'Exemple 1 (cristaux de granulométrie moyenne égale à 300 µm enrobés par de l'éthylcellulose).

Pour améliorer la stabilité de la composition, les excipients précédemment décrits sont modifiés, et la composition est la suivante :

| | |
|---|---|
| acide acétylsalicylique cristallisé encapsulé | 31,25 mg |
| acétate d'α-(dl)-tocophérol | 30,0 mg |
| vaseline épaisse | 60,0 mg |
| huile de vaseline | 38,75 mg |

Les valeurs indiquées ci-dessus correspondent à une capsule.

La tunique de la capsule a une composition identique à celle de l'Exemple 1.

Une étude de dégradation accélérée montre une excellente stabilité de la composition dans des conditions de conservation pendant 11 semaines sous humidité ambiante, à une température de 25°C, 37°C et 45°C, comme le montre le tableau ci-dessous. Les résultats sont exprimés en % du principe actif de départ. Les conditionnements utilisés pour cette étude sont identiques à ceux de l'Exemple 1.

| Produit de dégradation | Température | | |
|---|---|---|---|
| | 25°C | 37°C | 45°C |
| Acide salicylique | 0,13 | 0,17 | 0,21 |
| Tocophérol libre | 0,08 | 0,11 | 0,10 |

Ces résultats démontrent l'excellente stabilité de la composition de l'invention en utilisant comme excipient le mélange de vaselines ci-dessus.

### Exemple 4

On procède comme dans l'Exemple 3, mais en remplaçant le mélange d'huile de vaseline fluide et de vaseline épaisse par un mélange de triglycérides à chaînes moyennes et de triglycérides saturés.

Des résultats analogues à ceux de l'Exemple 3 sont obtenus, avec des taux de dégradation comparables dans l'essai de dégradation à 25°C, légèrement supérieurs dans l'essai à 37°C, et nettement supérieurs dans l'essai à 45°C, tous ces taux restant cependant inférieurs aux limites admises.

Dans les exemples ci-dessus, les opérations peuvent être mises en oeuvre sous azote pour diminuer encore la dégradation de l'acide acétylsalicylique.

### Exemple 5

On prépare plusieurs compositions identiques à celle décrite dans l'Exemple 1 mais en remplaçant l'acide acétylsalicylique cristallisé enrobé, successivement par les formes suivantes :
- micropulvérisée (non enrobée) de granulométrie moyenne égale à 90 µm,
- cristallisée (non enrobée) de granulométrie moyenne égale à 250 µm,
- cristallisée et enrobée par de l'éthylcellulose de granulométrie moyenne égale à 1000 µm,
ainsi que par le dérivé suivant :
- lysinate d'acide acétylsalicylique.

De plus, on fait varier les rapports acide acétylsalicylique / acétate de tocophérol de 1/7 à 2/1.

On prépare des compositions de la même manière à partir de la composition de l'Exemple 3.

On place ces formulations dans des conditions de dégradations accélérées (40°C sous humidité ambiante et 40°C sous 75% d'humidité relative). On dose à intervalles réguliers l'acide salicylique pour évaluer la dégradation des formulations.

Après 1 mois de test de stabilité, on constate d'une manière générale que la dégradation de l'acide acétylsalicylique est d'autant plus forte que sa concentration est plus faible dans la formule. Par exemple pour l'aspirine micropulvérisée, la teneur initiale en acide salicylique est de 0,15% m/m de l'acide acétylsalicylique. En un mois, elle augmente de 0,35% pour la formule la plus concentrée et de 2,5% pour la formule la plus diluée.

De même, on constate que les excipients de la formule jouent un rôle important dans la stabilité du produit. En particulier, l'emploi de vaselines améliore de manière connue la stabilité du produit.

Par contre, et de manière surprenante, les résultats montrent que la granulométrie n'est pas un élément fondamental de la stabilité. Les différences de dégradation entre les formulations à base d'aspirine micropulvérisée et cristallisée (non enrobées) ne sont pas significatives dans les conditions testées.

De même, il est surprenant de constater que l'emploi de lysinate d'acide acétylsalicylique conduit à des formulations très instables. En effet, la teneur en acide salicylique est aux environs de 6% m/m d'acide acétylsalicylique et, dans ce cas, le bénéfice d'une formulation à base de vaseline n'est pas évident.

### Exemple 6

En procédant comme dans l'Exemple 5, on prépare deux types de compositions avec les excipients décrits dans les exemples 1 et 3 mais en remplaçant, pour chacune des deux compositions, l'acétate de tocophérol par :
- succinate de tocophérol (sous forme de poudre sèche),
- nicotinate de tocophérol (sous forme pâteuse à température ordinaire),
- acétate de tocophérol (sous forme liquide à température ordinaire), selon l'invention.

Ces formulations sont soumises à des essais de dégradation accélérée comme décrit dans l'exemple 5.

| Excipients = triglycérides | % d'acide salicylique après 2 semaines à 40°C sous humidité ambiante |
|---|---|
| acétate de tocophérol | 0,4 % |
| succinate de tocophérol | 2,6 % |
| nicotinate de tocophérol | 4,7 % |

| Excipients = vaselines | % d'acide salicylique après 2 semaines à 40°C sous humidité ambiante |
|---|---|
| acétate de tocophérol | 0,3 % |
| succinate de tocophérol | 0,8 % |
| nicotinate de tocophérol | 1,5 % |

Les résultats, représentés dans les tableaux ci-dessus, démontrent que pour obtenir une formule stable associant de l'acide acétylsalicylique et du tocophérol, le tocophérol doit être préférentiellement sous forme d'acétate de tocophérol.

Dans tout ce qui précède, les quantités de tocophérol peuvent être exprimées soit en mg, soit en unités internationales (UI) en respectant les règles officielles de correspondance (Food and Nutrition Board, Recommended dietary allowances, 9ème édition, National Academy of Sciences, Washington D.C. (1980); Horwitt M.K., Amer. J. Clin. Nutr., 29 (1976) 569). Suivant ces règles, par exemple, 1 mg de dl-α-tocophérol est équivalent à 1,1 UI et 1 mg de d-α-tocophérol est équivalent à 1,49 UI.

## Revendications

1. Composition pharmaceutique stable à base d'acide acétylsalicylique et de tocophérol, pour l'administration par voie orale, **caractérisée en ce qu'**elle comprend des capsules contenant de l'acide acétylsalicylique sous forme cristalline ou pulvérisée, en mélange avec du tocophérol ou un de ses sels ou esters pharmaceutiquement acceptables sous forme huileuse.

2. Composition selon la revendications 1, **caractérisée en ce que** le tocophérol utilisé est l'α, le β-, le γ- ou le δ-tocophérol sous forme d, l ou dl.

3. Composition selon l'une quelconque des revendications 1 et 2, **caractérisée en ce que** l'ester de tocophérol est l'acétate de tocophérol.

4. Composition selon la revendication 1, **caractérisée en ce que** la granulométrie de l'acide acétylsalicylique sous forme cristalline ou pulvérisée est inférieure à 500 µm.

5. Composition selon l'une quelconque des revendications 1 et 4, **caractérisée en ce que** les grains d'acide acétylsalicylique sont encapsulés dans un dérivé cellulosique.

6. Composition selon l'une quelconque des revendications précédentes, **caractérisée en ce qu'**elle contient entre 20 et 300 mg d'acide acétylsalicylique et entre 20 et 600 mg de tocophérol par capsule.

7. Composition selon la revendication 1, **caractérisée en ce que** les capsules contiennent au moins un excipient choisi parmi des triglycérides et des vaselines.

8. Composition selon la revendication 1, **caractérisée en ce que** la paroi des capsules est à base de gélatine et/ou de glycérol.

9. Composition selon la revendication 1, **caractérisée en ce que** les capsules sont conditionnées dans un composite aluminium / chlorure de polyvinyle.

## Patentansprüche

1. Stabile pharmazeutische Zusammensetzung auf der Basis von Acetylsalicylsäure und Tocopherol zur oralen Verabreichung, **dadurch gekennzeichnet, dass** sie Kapseln umfasst, die Acetylsalicylsäure in kristalliner Form oder in Pulverform im Gemisch mit Tocopherol oder einem pharmazeutisch annehmbaren Salz oder Ester davon in Form eines Öls enthalten.

2. Zusammensetzung nach Anspruch 1, **dadurch gekennzeichnet, dass** das verwendete Tocopherol α-, β-, γ- oder δ-Tocopherol in d-, l- oder dl-Form ist.

3. Zusammensetzung nach einem der Ansprüche 1 oder 2, **dadurch gekennzeichnet, dass** der Tocopherolester Tocopherolacetat ist.

4. Zusammensetzung nach Anspruch 1, **dadurch gekennzeichnet, dass** die Korngröße der Acetylsalicylsäure in kristalliner oder Pulverform unter 500 µm liegt.

5. Zusammensetzung nach einem der Ansprüche 1 bis 4, **dadurch gekennzeichnet, dass** die Acetylsalicylsäure-Körner in ein Cellulosederivat eingekapselt sind.

6. Zusammensetzung nach einem der vorangehenden Ansprüche, **dadurch gekennzeichnet, dass** sie zwischen 20 und 300 mg Acetylsalicylsäure und zwischen 20 und 600 mg Tocopherol pro Kapsel enthält.

7. Zusammensetzung nach Anspruch 1, **dadurch gekennzeichnet, dass** die Kapseln zumindest einen aus Triglyceriden und Vaselinen ausgewählten Arzneimittelträger enthalten.

8. Zusammensetzung nach Anspruch 1, **dadurch gekennzeichnet, dass** die Hülle der Kapseln auf Gelatine und/oder Glycerin basiert.

9. Zusammensetzung nach Anspruch 1, **dadurch gekennzeichnet, dass** die Kapseln in einem Verbund aus Aluminium und Polyvinylchlorid verpackt sind.

## Claims

1. Stable pharmaceutical composition based on acetylsalicylic acid and tocopherol, for oral administration, **characterized in that** it comprises capsules containing acetylsalicylic acid in crystalline or powdered form in admixture with tocopherol or one of its pharmaceutically acceptable salts or esters in oily form.

2. Composition according to Claim 1, **characterized in that** the tocopherol used is α-, β-, γ- or δ-tocopherol in d, l or dl form.

3. Composition according to any of Claims 1 and 2, **characterized in that** the tocopherol ester is tocopherol acetate.

4. Composition according to Claim 1, **characterized in that** the particle size of the acetylsalicylic acid in crystalline or powdered form is less than 500 µm.

5. Composition according to either of Claims 1 and 4, **characterized in that** the particles of acetylsalicylic acid are encapsulated in a cellulose derivative.

6. Composition according to any one of the preceding claims, **characterized in that** it contains between 20 and 300 mg of acetylsalicylic acid and between 20 and 600 mg of tocopherol per capsule.

7. Composition according to Claim 1, **characterized in that** the capsules contain at least one excipient chosen from triglycerides and petroleum jellies.

8. Composition according to Claim 1, **characterized in that** the wall of the capsules is based on gelatin and/or glycerol.

9. Composition according to Claim 1, **characterized in that** the capsules are packaged in an aluminium/polyvinyl chloride composite.
